(19) Europäisches Patentamt
European Patent Office
Office européen des brevets

(11) **EP 1 886 667 A1**

(12) **EUROPEAN PATENT APPLICATION**

(43) Date of publication:
**13.02.2008 Bulletin 2008/07**

(51) Int Cl.:
*A61K 9/107* (2006.01)     *A61K 47/10* (2006.01)

(21) Application number: **06016490.2**

(22) Date of filing: **08.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **The Jordanian Pharmaceutical Manufacturing Co.**
**Ltd.**
**11710 Naor (JO)**

(72) Inventors:
• **Badwan, Adnan**
**Amman 11185 (JO)**

• **Al-Remawi, Mayyas**
**Russeifa 13713 (JO)**
• **Fakherddin, Mohammad**
**Amman 11821 (JO)**
• **Al-Taani, Mohammad**
**Irbid (JO)**

(74) Representative: **Winkler, Andreas Fritz Ernst**
**FORRESTER & BOEHMERT**
**Pettenkoferstrasse 20-22**
**80336 München (DE)**

(54) **Microemulsified drug formulation**

(57)     The present invention relates to a microemulsified drug formulation comprising at least one surfactant, at least one co-surfactant, an oil phase, water and at least one drug selected from classes II-IV of the biopharmaceutical classification system (BCS).

**EP 1 886 667 A1**

**Description**

**Field of the invention**

The present invention relates to a microemulsified drug formulation which relates to the field of enhancement of absorption kinetics of poorly absorbable drugs.

**Background**

The absorption and consequently the bioavailability of an oral solid dosage form depend on two main processes, drug dissolution and permeation.

Drug dissolution is the process in which the drug is released, made available in solution and ready to be absorbed. Physicochemical properties of a drug such as solubility as well as the gastrointestinal environment are the most important parameters affecting dissolution. Drug permeability commences following the conversion of the solid drug into solution. Permeability is the ability of the drug to penetrate across a membrane into the systemic circulation. The extent of permeation and ultimately absorption also depends upon the physicochemical properties of the drug and blood perfusion.

Drugs can be classified according to the biopharmaceutical classification system (BCS) into four classes. [G. Amidon, H. Lennemas, V. Shah, and J. Crison,. A theoretical basis for a biopharmaceutic drug classification: The correlation of in vitro drug product dissolution and in vivo bioavailabilty. Pharm. Res., 12, pp 413-419, 1995; Jennifer B.Dressman, Gordon L. Amidone, Chritos Reppas, and Vinod P.Shah. Dissolution testing as a prognostic tool for oral drug absorption: Immediate release dosage forms. Pharmaceutical research. 1998; 15: 11-20].

Thus, biopharmaceutical drug classification is a fundamental guideline classification of drugs based on the solubility and permeability, as shown in table 1.

Table 1: Biopharmaceutics Drug Classification

| Class | Solubility | Permeability |
| --- | --- | --- |
| I | High | High |
| II | Low | High |
| III | High | Low |
| IV | Low | Low |

Class I includes drugs that are water soluble and gastrointestinal tract permeable. This class does not suffer from absorption or permeation problems that may affect oral drug bioavailability.

While classes II, III and IV contain drugs having problems in solubility and/or permeability that may be reflected on their bioavailability in the blood after the drug is taken orally. Classes II, III and IV form approximately 80% of the drugs available in the market. Table 2 summarizes examples of drugs related to these classes.

Table (2): some examples of drugs related to classes II, III and IV

| Class II | Class III | Class IV |
| --- | --- | --- |
| Artemether | Abacavir | Nelfinavir Mesylate |
| Carbamazepine | Acetylsalicylic Acid | Furosemide |
| Dapsone | Allopurinol | Albendazole |
| Efavirenz | Atropine Sulfate | Acetazolamide |
| Folic Acid | Biperiden Hydrochloride | Azathioprinei |
| Glibenclamide | Captopril | Didancosine |
| Griseofulvin | Chloramphenicol | Indinavir |
| Haloperidol | Cimetidine | |
| Ibuprofen | Colchicines | |
| Phenytoin Sodium | Ergometrine | |
| | Ethambulol Hydrochloride | |
| | Hydralazine | |
| | Hydrochlorothaizide | |

(continued)

| Class II | Class III | Class IV |
|---|---|---|
| | Lamivudine | |
| | Levamisole | |
| | Metformin Hydrochloride | |
| | Propylthiouracil | |

The vast majority of commercially available oral formulations are solid dosage forms such as tablets or capsules, but there are many solubilized oral formulations such as oral solutions, syrups, elixirs, and solutions filled into soft or hard capsules. The reasons for pursuing a solubilized oral formulation include enhancing the oral bioavailability of poorly water-soluble molecule, a measurable formulation for dose modification, a formulation for patients who cannot swallow tablets or capsules.

Many strategies have been applied to develop a bioactive oral formulation to be delivered as oral tablets, suspension and soft gelatin capsules. The strategies include the co-solvent system, surfactants (micelles), pH adjustment, cyclodextrine complexation, liposomes and micro-emulsions. Micro-emulsion systems exhibit a major advantage on enhancing the permeability of many drugs.

[Physician's disk reference, 57th ed., medical economics company, Inc., Montvale, NJ, 2003; Physician's disk reference, 54th ed., medical economics company, Inc., Montvale, NJ, 2000; Product literature, Gattefosse Corp., 372 Kinderkamack Road, Westwood, NJ 07675; N.H.Shah, M.T. Carvajal, C.I. Patel, M.H.Infeld, and A.W.Malick. Self emulsifying drug delivery systems (SEDDS) with polyglycolized glycerides for improving in vitro dissolution and oral absorption of lipophilic drugs. Int.J.Pharm. 106: 15-23 (1994); T.Loftsson and M.E. Brewster. Pharmacetical applications of cyclodextrins I. Drug solubilization and stabilization. J.Pharm.Sci. 85: 1017-1024 (1996); T.Irie and K. Uekama. Pharamaceutical applications of cyclodextrins. III. Toxicology issues and safty evaluation. J. Pharm.Sci. 86:147-162 (1997); Brime et al., Amphotericin B in oil-water-lecithin-based microemulsions: formulation and toxicity evaluation. Journal of pharmaceutical sciences 91:1178-1184 (2002); S.Tenjarla. Microemulsions: an overview and pharmaceutial applications. Crit. Rev. Ther. Drug carrier Syst. 16: 461-521 (1999); M. Jayne Lawrence, Gareth D. Rees. Microemulsion-based media as a novel drug delivery systems. Advanced drug delivery reviews. 45: 89-121 (2000)].

It is an object of the present invention to provide a microemulsified drug formulation overcoming the difficulties of the prior art, especially to provide a drug formulation for poorly bioavailable drugs to provide improved bioavailability.

This object is achieved by a microemulsified drug formulation according to claim 1. Preferred embodiments are disclosed in the sub-claims.

The oil phase as utilized in the microemulsified drug formulation according to the present invention may be any phase that contains any organic material with a solubility parameter below 15, thus, this can be literally any fatty acid, triglyceride, ether, alcohol, etc.

Surprisingly it was found that a completely solubilized formulation of a drug of classes II-IV of the biopharmaceutical classification system showed higher bioavailability compared to a reference formulation present in the market.

The present invention discloses a microemulsified drug system which is formed substantially by a surfactant and a co-surfactant. The cosurfactant is added to the microemulsified system to decrease the amount of surfactant consumed in the final formulation. This will minimize the known irritant effect of the surfactants. In addition, the cosurfactant acts as permeability enhancer. This means the microemulsified system is a balanced system that contains the main permeability enhancement mechanisms namely, hydration, high drug concentrations and permeability enhancers.

The surfactant is a surface active compound or other material that has the ability to alter or reduce surface tension of materials that are dissolved into it. Suitable surfactants include polyethoxylated castor oil, ethoxylated soribitans, sorbitan fatty acid esters, ethoxylated sorbitol and sorbitol esters, ethoxylated fatty acids, polyethylene glycol fatty acids esters, ethoxylated alcohols and ethoxylated triglycerides. Most polyethoxylated vegetable oils can be used as the oil surfactant necessary to the practice of the invention.

The cosurfactant is a product, which has the ability to dissolve in oil, alcohol or a water phase. Virtually any alcohol except methanol may serve as the co-surfactant. Isopropyl alcohol is one co-surfactant of choice. Other suitable co-surfactants include glycerin propylene glycol, polyethylene glycol, and other alcohols such as cetyl alcohol.

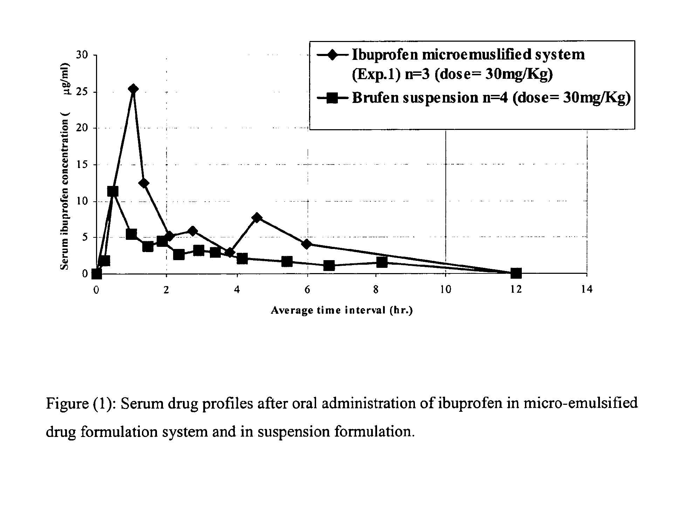

Additional advantages and features of the present invention will become apparent in the following detailed description of the examples with reference to the accompanying drawing, wherein figure 1 illustrates serum drug profiles after oral administration of ibuprofen in microemulsified drug formulation and in suspension formulation.

The following examples are provided utilizing ibuprofen, hydrochlorotiazid and furosemid taken as examples for classes II, III and IV, respectively. The formulation evaluation is illustrated in the following examples.

**Examples**

**Example 1**

Different formulations of microemulsified system were prepared as shown in Table 3. These formulations provide a microemulsified system. The formulation is generally comprises about 0.1 to 95%, more particularly 15 to 70%, water; 0.1 to 90%, more particularly 10to 50%,surfactant PEG-20 glycerol laurate; 0.1 to 95%, more particularly 5% to 40%, ethoxydiglycol; 0.1 to 95%, more particularly 1% to 70% oil phase such as isopropylmyristate; and 0.001 to 80%, more particularly 5% to 50% , active material. Table 3 shows the most preferable compositions.

Table (3): Summary of the most promising microemulsified system formulas containing Ibuprofen (class II)

| Trial # | % w/w | | | | |
|---|---|---|---|---|---|
| | Ibuprofen | De-ionized water | PEG-20 glycerol laurate | Ethoxy diglycole | Isopropyl myristate |
| 1 | 9.8 | 37.45 | 30.59 | 14.03 | 8.13 |
| 3 | 6.26 | 59.99 | 19.58 | 9 | 5.17 |
| 4 | 7.91 | 49.42 | 24.77 | 11.36 | 6.54 |
| 5 | 9.38 | 40.05 | 29.35 | 13.47 | 7.75 |

The procedure utilized in the preparation is as follows: at given amounts of surfactant, oil and aqueous phase, the creamy emulsion that results is further titrated with a suitable cosurfactant, with continuous mixing using a magnetic stirrer, until a clear emulsion is obtained. The titration is done by weight (not by volume) since all the constituents were previously quantitated by weight. The clear microemulsified system obtained represents the end point.

**Example 2**:

The relative bioavailability for ibuprofen microemulsified system formulated in example 1, Table 3, trial # 1 was also done using rabbit model taking Brufen® suspension as a reference oral dosage form. In all experiments, the dose of ibuprofen taken by the animal has been set at 30mg/kg.

The analysis of samples was done using high performance liquid chromatography, the mobile phase for bioavailability studies in rabbits was prepared as follow:

About 34.84g of anhydrous dipotassium hydrogen phosphate, accurately weighted, was dissolved in 1 L de-ionized water and then adjusted with phosphoric acid to pH 6.5. The mobile phase consists of a filtered and degassed mixture of buffer: water: acetonitrile at a volume ratio 10:55:35, respectively.

Chromatographic system:

| | |
|---|---|
| Flow rate | 1ml/min. |
| Loop injector volume | 100 $\mu$l. |
| Wavelength | 220nm |
| Column | C18, BDS (Type B silica, Base Deactivated Silica), end-capped, 250*4.6mm, 5$\mu$. type Hypersil. |

Standards preparation:

A series of standards were prepared. 20mg ibuprofen was weighted accurately in 100 ml volumetric flask. Then 20 ml of methanol was added and the sample was sonicated in the ultrasound sonicator until ibuprofen dissolved completely. The volume was completed to 100 ml with water. A series of dilutions were prepared from this standard, using the diffusion buffer.

Biological samples preparation:

2 ml of blood was withdrawn from the rabbits at a time interval. The samples were left for 5 minutes at room temperature, to allow coagulation process, then centrifuged at 5000 rpm for 10 minutes. The supernatant (i.e., the serum) was then separated in another eppendorf tube closed well and deep-freezed for the second day (i.e., the day of analysis). 200 $\mu$l of the serum samples was withdrawn, using a 200 $\mu$l micropipette , into eppendorf tube. Then 800 $\mu$l methanol was added to the samples using 1000 $\mu$l micropipette. The samples were shaken for 30 seconds using a vortex mixer and then centrifuged at 4000 rpm. 100 $\mu$l of the supernatant was injected directly into the column (pre-column is recommended).

Figure (1) shows serum drug profiles after oral administration of ibuprofen to rabbits (30 mg/kg) in the microemulsified system of the present invention compared to that of a commercial reference product (Brufen Suspension) in suspension formulation.

Table 4 summarizes the relative bioavailability based on AUC and Cmax. Also it contains individual tmax, and AUC of the inventive formula (test) and the reference (commercial product). Results indicate a higher relative bioavailability for ibuprofen using the inventive micremulsified system over a commercial reference product. This may indicate the superiority of this system in increasing drug ultimate absorption by increasing solubilization and/or permeation through the GIT.

Where, relative bioavalability (RB) was determined based on pharmacokinetic parameter ratios of AUC and Cmax (Test formula/Reference formula) of according to following equations:

$$RB = AUC_{test} / AUC_{reference}$$

$$RB = Cmax_{test} / Cmax_{reference}$$

[L. Shargel and A. Yu. Applied Biopharmaceutics and Pharmacokinetics, Appleton Century Crofts, Norwalk, Connecticut, second edition, pp129, 1985]

Table (4): Relative bioavailability of ibuprofen microemulsified system experiment 1 vs. oral Brufen suspension.

| Delivery system | RB AUC | RB Cmax | Cmax ($\mu$g/ml) | Duration (hr.) | Time to reach Cmax (hr.) | AUC ($\mu$g/ml *hr.) |
|---|---|---|---|---|---|---|
| Brufen® suspension | Reference | Reference | 11.4 | 12 | 0.5 | 25.9 |
| Exp.1 (Ibuprofen oral microemulsified system) | 228.9% | 221.9% | 25.3 | 12 | 1 | 59.3 |

**Example 3**:

Different formulations of microemulsified system were prepared using hydrochlorothiazide. These formulas provide a microemulsified system. The formula is generally comprises about 0.1 to 95%, more particularly 5to 15%, water; 0.1 to 90%, more particularly 15to 25%,surfactant PEG-40 glycerol recinoleate; 0.1 to 95%, more particularly 25% to 35%, propylene glycol monocaprylate; 0.1 to 95%, more particularly 35% to 50% oil phase such as Octyl dodecyl myristate; and 0.001 to 80%, more particularly 0.001% to 0.01% , active material. Table 4 shows the most preferable composition

Table (4): Summary of the most promising microemulsified system formulas containing Hydrochlorothiazide (class III)

| % w/w | | | | |
|---|---|---|---|---|
| Hydrochlorothiazide | De-ionized water | PEG-40 glycerol ricinoleate | Propylene glycol mono caprylate | Octyl dodecyl myristate |
| 0.0014 | 14 | 19 | 30 | 37 |

**Example 4:**

Different formulations of microemulsified system were prepared using furosemide. These formulas provide a microemulsified system

The formula is generally comprises about 0.1 to 95%, more particularly 5to 15%, water; 0.1 to 90%, more particularly 15to 25%,surfactant PEG-8 dilaurate; 0.1 to 95%, more particularly 25% to 35%, PEG-2 lauryl ether; 0.1 to 95%, more particularly 35% to 50% oil phase such as medium chain glycerides; and 0.001 to 80%, more particularly 0.1% to 1% , active material. Medium chain glycerides are glycerides with a fatty chain that contains 6-8 carbon atoms (e.g. coconut oil). Table 5 shows the most preferable composition

Table (5): Summary of the most promising microemulsified system formulas containing Furosemide (class IV)

| % w/w | | | | |
|---|---|---|---|---|
| Furosemide | De-ionized water | PEG-8 dilaurate | PEG-2 lauryl ether | Medium chain glycerides |
| 0.2 | 13 | 23 | 32 | 31.8 |

The features disclosed in the foregoing description, in the claims and/or in the accompanying drawings may, both separately and in any combination thereof, be material for the realizing the invention in diverse forms thereof.

**Claims**

1. Microemulsified drug formulation comprising at least one surfactant, at least one co-surfactant, an oil phase, water and at least one drug selected from classes II-IV of the biopharmaceutical classification system (BCS).

2. Microemulsified drug formulation according to claim 1, wherein the surfactant is present in an amount of 0.1 to 95 weight percent, preferably 10 to 50 weight percent, more preferably 15 to 35 weight percent of surfactant, based on the total weight of the formulation.

3. Microemulsified drug formulation according to claim 1 or 2, wherein the co-surfactant is present in an amount of 0.1 to 95 weight percent, preferably 5 to 40 weight percent, more preferably 5 to 35 weight percent, based on the total weight of the formulation.

4. Microemulsified drug formulation according to any of the preceding claims, wherein the oil phase is present in an amount of 0.1 to 95 weight percent, preferably 1 to 70 weight percent, more preferably 5 to 50 weight percent, based on the total weight of the formulation.

5. Microemulsified drug formulation according to any of the preceding claims, wherein water is present in an amount of 0.1 to 95 weight percent, preferably 10 to 70 weight percent, more preferably 10 to 50 weight percent, based on the total weight of the formulation.

6. Microemulsified drug formulation according to any of the preceding claims, wherein the surfactant is selected from the group consisting of polyethoxylated castor oil, ethoxylated sorbitans, sorbitan fatty acid esters, ethoxylated sorbitol and sorbitol esters ethoxylated fatty acids, polyethylene glycol fatty acid esters, ethoxylated alcohols, ethoxylated triglycerides and polyethoxylated vegetable oils, preferably PEG-20 glycerol laurate, PEG-40 glycerol recinoleate and PEG-8 dilaurate.

7. Microemulsified drug formulation according to any of the preceding claims, wherein the co-surfactant is selected from alcohols, preferably isopropyl alcohol, glycerin, propylene glycol, polyethylene glycol and/or cetyl alcohol.

**8.** Microemulsified drug formulation according to any of the preceding claims, wherein the oil phase is selected from the group consisting of isopropylmyristate, octyldodecylmyristate and medium chain glycerides.

**9.** Microemulsified drug formulation according to any of the preceding claims, wherein the drug is present in an amount of 0.001 to 80 percent by weight, preferably 0.001 to 50 weight percent, more preferably 0.001 to 10 weight percent, based on the total weight of the formulation.

Figure (1): Serum drug profiles after oral administration of ibuprofen in micro-emulsified drug formulation system and in suspension formulation.

**European Patent Office**

**PARTIAL EUROPEAN SEARCH REPORT**

which under Rule 45 of the European Patent Convention shall be considered, for the purposes of subsequent proceedings, as the European search report

Application Number

EP 06 01 6490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN, H. ET AL: "Microemulsion-based gydrogel formulation of ibuprofen for topical delivery" INTERNATIONAL JOURNAL OF PHARMACEUTICS, no. 3015, 5 April 2006 (2006-04-05), pages 52-58, XP002406070 * page 52, column 1, lines 1-5 * * page 53, column 1, lines 20-40 * ----- | 1-9 | INV. A61K9/107 A61K47/10 |
| X | US 2002/032171 A1 (CHEN FENG-JING [US] ET AL) 14 March 2002 (2002-03-14) * page 5; table 1 * * page 8; tables 2,3 * * page 21, paragraph 145 * * page 24, paragraph 164-166 * * page 33; table 25 * ----- -/-- | 1-9 | |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 11 January 2007 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**   **PARTIAL EUROPEAN SEARCH REPORT**   Application Number

EP 06 01 6490

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 2006/061827 A (NUTRALEASE LTD [IL]; GARTI NISSIM [IL]; ASERIN ABRAHAM [IL]; KOGAN ANN) 15 June 2006 (2006-06-15)<br>* page 2, line 1 *<br>* page 6, lines 27-29, 17-33 *<br>* page 7, lines 1-8 *<br>* page 10, lines 18,19 *<br>* page 12, lines 1-25 *<br>* page 36; claims 1-7 *<br>* page 37; claims 14-16 *<br>* page 38; claims 17-21 *<br>* page 39; claim 22 *<br>----- | 1-9 | |
| X | WO 02/074282 A (ASTRAZENECA AB [SE]; SIEKMANN BRITTA [SE]; THORING BARBRO [SE]) 26 September 2002 (2002-09-26)<br>* page 1, lines 1-7,18-22 *<br>* page 7, lines 5-7 *<br>* page 10, lines 1-24 *<br>* page 11, lines 1-8,13,14,20-24 *<br>* page 15; example 1 *<br>* page 29; claim 15 *<br>* page 30; claim 17 *<br>----- | 1,2,4-9 | **TECHNICAL FIELDS SEARCHED** (IPC) |
| X | WO 03/045357 A (TRANSFORM PHARMACEUTICALS INC [US]; CHEN HONGMING [US]; ZHANG ZHONG [U) 5 June 2003 (2003-06-05)<br>* page 4, lines 4-6,19-21 *<br>* page 8, lines 21-36 *<br>* page 9, lines 1-27 *<br>* page 14, lines 21-34 *<br>* page 15 *<br>* page 16, lines 1-22 *<br>* page 22, lines 34,35 *<br>* page 23, lines 1,4,22 *<br>* page 30; claims 17,18 *<br>* page 31; claims 19-21 *<br>* page 32; claim 22 *<br>----- | 1-9 | |

-/--

EPO FORM 1503 03.82 (P04C10)

**European Patent Office**

**PARTIAL EUROPEAN SEARCH REPORT**

Application Number

EP 06 01 6490

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | WO 2005/037249 A (HANMI PHARM IND CO LTD [KR]; WOO JONG SOO [KR]; JUNG SI YOUNG [KR]; KI) 28 April 2005 (2005-04-28)<br>* page 1, lines 6-9,28,29 *<br>* page 3, lines 12-23 *<br>* page 4, lines 1-25 *<br>* page 5, lines 1-18 *<br>----- | 1,6-8 | |
| X | WO 02/09764 A (BYRON BASSETT LTD [NZ]; RAZZAK MAJID HAMEED ABDUL [NZ]; WATNASIRICHAIK)<br>7 February 2002 (2002-02-07)<br>* page 1, lines 21-25 *<br>* page 2, lines 29-32 *<br>* page 3, lines 6,7,11,12,19,20 *<br>* page 12; claims 1,2,5-7,10-12 *<br>----- | 1-9 | TECHNICAL FIELDS SEARCHED (IPC) |
| X | WO 2005/065652 A1 (FRIEDMAN DORON [IL])<br>21 July 2005 (2005-07-21)<br>* page 5, paragraph 5 *<br>* page 6, paragraph 1 *<br>* page 11, paragraph 4 *<br>* page 13, paragraphs 4,5 *<br>* page 14, paragraph 4 *<br>* page 16, paragraph 2 *<br>* page 23, paragraph 3 *<br>----- | 1,6-8 | |

EPO FORM 1503 03.82 (P04C10)

**European Patent Office**

**INCOMPLETE SEARCH
SHEET C**

Application Number

EP 06 01 6490

```
Claim(s) searched incompletely:
        1-9

Reason for the limitation of the search:

The present claim 1 relates to an extremely large number of possible
compounds. Support and disclosure in the sense of Article 84 and 83 EPC
is to be found however for only a very small proportion of the compounds.
The non-compliance with the substantive provisions is to such an extent,
that a meaningful search of the whole claimed subject-matter of the claim
could not be carried out (Rule 45 EPC and Guidelines B-VIII, 3). The
extent of the search was consequently limited.
```

## ANNEX TO THE EUROPEAN SEARCH REPORT
## ON EUROPEAN PATENT APPLICATION NO.

EP 06 01 6490

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | | Publication date | Patent family member(s) | | Publication date |
|---|---|---|---|---|---|
| US 2002032171 | A1 | 14-03-2002 | NONE | | |
| WO 2006061827 | A | 15-06-2006 | NONE | | |
| WO 02074282 | A | 26-09-2002 | BR 0207760 | A | 01-06-2004 |
| | | | CA 2435825 | A1 | 26-09-2002 |
| | | | CN 1496253 | A | 12-05-2004 |
| | | | EP 1370239 | A1 | 17-12-2003 |
| | | | JP 2004523577 | T | 05-08-2004 |
| | | | MX PA03007093 | A | 18-11-2003 |
| | | | NO 20034026 | A | 11-11-2003 |
| | | | US 2004096494 | A1 | 20-05-2004 |
| | | | ZA 200306282 | A | 23-11-2004 |
| WO 03045357 | A | 05-06-2003 | AU 2002357012 | A1 | 10-06-2003 |
| WO 2005037249 | A | 28-04-2005 | CN 1870975 | A | 29-11-2006 |
| | | | EP 1682088 | A1 | 26-07-2006 |
| | | | KR 20050038224 | A | 27-04-2005 |
| WO 0209764 | A | 07-02-2002 | US 2003180350 | A1 | 25-09-2003 |
| WO 2005065652 | A1 | 21-07-2005 | CA 2547712 | A1 | 21-07-2005 |
| | | | EP 1706100 | A1 | 04-10-2006 |

For more details about this annex : see Official Journal of the European Patent Office, No. 12/82

# EP 1 886 667 A1

### Non-patent literature cited in the description

- **G. AMIDON ; H. LENNEMAS ; V. SHAH ; J. CRISON.** A theoretical basis for a biopharmaceutic drug classification: The correlation of in vitro drug product dissolution and in vivo bioavailabilty. *Pharm. Res,* 1995, vol. 12, 413-419
- **JENNIFER B.DRESSMAN ; GORDON L. AMIDONE ; CHRITOS REPPAS ; VINOD P.SHAH.** Dissolution testing as a prognostic tool for oral drug absorption: Immediate release dosage forms. *Pharmaceutical research,* 1998, vol. 15, 11-20
- Physician's disk reference. medical economics company, Inc, 2003
- Physician's disk reference. medical economics company, Inc, 2000
- Product literature. Gattefosse Corp
- **N.H.SHAH ; M.T. CARVAJAL ; C.I. PATEL ; M.H.INFELD ; A.W.MALICK.** Self emulsifying drug delivery systems (SEDDS) with polyglycolized glycerides for improving in vitro dissolution and oral absorption of lipophilic drugs. *Int.J.Pharm.,* 1994, vol. 106, 15-23
- **T.LOFTSSON ; M.E. BREWSTER.** Pharmacetical applications of cyclodextrins I. Drug solubilization and stabilization. *J.Pharm.Sci.,* 1996, vol. 85, 1017-1024
- **T.IRIE ; K. UEKAMA.** Pharamaceutical applications of cyclodextrins. III. Toxicology issues and safty evaluation. *J. Pharm.Sci.,* 1997, vol. 86, 147-162
- **BRIME et al.** Amphotericin B in oil-water-lecithin-based microemulsions: formulation and toxicity evaluation. *Journal of pharmaceutical sciences,* 2002, vol. 91, 1178-1184
- **S.TENJARLA.** Microemulsions: an overview and pharmaceutial applications. *Crit. Rev. Ther. Drug carrier Syst.,* 1999, vol. 16, 461-521
- **M. JAYNE LAWRENCE ; GARETH D. REES.** Microemulsion-based media as a novel drug delivery systems. *Advanced drug delivery reviews,* 2000, vol. 45, 89-121
- **L. SHARGEL ; A. YU.** Applied Biopharmaceutics and Pharmacokinetics. Appleton Century Crofts, 1985, 129